# EUROPÄISCHE PATENTSCHRIFT

(11) **EP 3 393 635 B1**
(45) Veröffentlichungstag und Bekanntmachung des Hinweises auf die Patenterteilung: **11.11.2020**
(21) Anmeldenummer: 16834020.6
(22) Anmeldetag: 23.12.2016
(51) Int. Cl.: B01D 65/10, A61M 1/16

(54) **VERFAHREN UND VORRICHTUNG ZUR ÜBERPRÜFUNG EINES DIALYSATORS AUF DAS VORHANDENSEIN EINES LECKS**
METHOD AND DEVICE FOR CHECKING A DIALYZER FOR THE PRESENCE OF A LEAK
PROCÉDÉ ET DISPOSITIF DE VÉRIFICATION DE LA PRÉSENCE D'UNE FUITE SUR UN DIALYSEUR

(30) Priorität: 23.12.2015 DE 102015016842
(43) Veröffentlichungstag der Anmeldung: 31.10.2018
(73) Patentinhaber: Fresenius Medical Care Deutschland GmbH, 61352 Bad Homburg (DE)
(72) Erfinder: SPICKERMANN, Reiner, 97535 Wasserlosen-Burghausen (DE); WIESEN, Gerhard, 61352 Bad Homburg (DE)
(74) Vertreter: Herrmann, Uwe
(86) Internationale Anmeldenummer: PCT/EP2016/002182
(87) Internationale Veröffentlichungsnummer: WO 2017/108196

(56) Entgegenhaltungen:
- DE-A1- 3 442 744
- DE-A1-102008 005 516
- DE-C1- 3 923 078
- JP-A- 2003 190 747
- US-A- 5 863 421

## Beschreibung

Die vorliegende Erfindung betrifft ein Verfahren zur Überprüfung eines Dialysators auf das Vorhandensein eines Lecks in der semipermeablen Dialysatormembran, die den Innenraum des Dialysators in wenigstens eine Blutkammer und in wenigstens eine Dialysatkammer trennt, wobei die Blutkammer mit einem blutseitigen Leitungssystem in Fluidverbindung steht, das während der Behandlung mit dem Gefäßsystem eines Patienten verbunden ist, und wobei die Dialysatkammer mit einem dialysatseitigen Leitungssystem in Fluidverbindung steht, so dass während der Blutbehandlung das Blut das blutseitige Leitungssystem und die Blutkammer und die Dialysierflüssigkeit die Dialysatkammer und das dialysatseitige Leitungssystem durchströmt.

Insbesondere Microlecks in der Membran des Dialysators, die eine Leckrate von kleiner als 0,5 ml/min aufweisen, sind mit bisher bekannten Verfahren nicht erkennbar. Ein Leck in der Membran eines Dialysators, durch das Blut aus der Blutkammer in die Dialysatkammer des Dialysators übertritt und somit in das dialysatseitige Leitungssystem eines Dialysegerätes gelangt, ist während einer laufenden Behandlung zwar unproblematisch, erfordert aber vor einer nachfolgenden Dialysebehandlung die Desinfektion des Dialysegerätes, um eine Kreuzkontamination bzw. eine Kontamination des nachfolgend behandelten Patienten zu verhindern.

Aus der US 6,804,991 B2 ist ein Verfahren bekannt, bei dem im Fluidsystem eines Blutbehandlungsgerätes eine Leckage erkannt werden kann. Das Verfahren basiert auf der Messung einer Druckveränderung während der Behandlung des Patienten.

Die WO 2013/017236 A1 betrifft ein Verfahren zur Überprüfung der Funktion von medizinischen Funktionseinrichtungen, wie beispielsweise von extrakorporalen Blutschläuchen. Das Verfahren umfasst den Druckaufbau sowie die Messung der Druckveränderung über die Zeit. Das Verfahren der WO 2013/017236 A1 wird vor Aufnahme der Behandlung eines Patienten durchgeführt.

Die US 8,241,237 B2 betrifft ein Verfahren zur Druckmessung in einer blutführenden Leitung. Zur Druckmessung dient ein Sensor, der durch eine spezielle Filteranordnung vor direktem Kontakt mit dem Blut des Patienten geschützt wird. Sofern nach der Blutbehandlung eine Fehlfunktion des Sensors festgestellt wird, wird der Sensor oder dessen defekte Teile ausgetauscht. Aus der US5863421 ist ein Unversehrtheitstest der Fasern des Dialysators bekannt, um sicherzustellen, daß der Dialysator keine Lecks aufweist. Dieser Test wird unter Luftdruck durchgeführt.

Der vorliegenden Erfindung liegt die Aufgabe zugrunde, ein Verfahren und eine Vorrichtung bereitzustellen, mittels dessen/derer ein Leck und insbesondere ein Microleck der Membran eines Dialysators besonders exakt ermittelbar ist.

Diese Aufgabe wird durch ein Verfahren mit den Merkmalen des Anspruchs 1 und mit einer Vorrichtung mit den Merkmalen des Anspruchs 8 gelöst.

Das Verfahren gemäß der Erfindung umfasst die folgenden Schritte:
a) Entleeren der Blutkammer oder der Dialysatkammer von Blut bzw. von Dialysierflüssigkeit und Beibehalten der Flüssigkeit (Blut oder Dialysat) in der nicht entleerten Dialysat- oder Blutkammer,
b) Aufbauen eines Testdruckes in der entleerten Blutkammer oder in der entleerten Dialysatkammer und
c) Messen des Druckanstiegs in der nicht entleerten Blutkammer oder Dialysatkammer oder in dem mit diesen jeweils in Fluidverbindung stehenden Leitungssystem,
wobei das gesamte Verfahren, einschließlich die Schritte a) bis c) im Anschluss an die Blutbehandlung des Patienten und im Anschluss an das Abklemmen des Patienten von dem blutseitigen Leitungssystem durchgeführt werden.

Erfindungsgemäß ist somit vorgesehen, die Blutkammer oder Dialysatkammer bzw. das gesamte mit diesen jeweils in Fluidverbindung stehende Leitungssystem, d.h. das blutseitige oder das dialysatseitige Leitungssystem zu entleeren, in der auf diese Weise entleerten Kammer bzw. Leitungssystem einen Druck aufzubauen und sodann den Druckverlauf über die Zeit zu messen.

Der Begriff "entleert" bezieht sich im Rahmen der vorliegenden Erfindung darauf, dass die Flüssigkeit entleert wurde, d.h. dass Blut aus der Blutkammer bzw. aus dem blutseitigen Leitungssystem oder Dialysierflüssigkeit aus der Dialysatkammer bzw. aus dem dialysatseitigen Leitungssystem vollständig oder teilweise entfernt wurde.

Des Weiteren wird darauf hingewiesen, dass die Begriffe "ein" oder "eine" nicht zwingend auf genau eines der fraglichen Elemente hinweisen, sondern auch eine Mehrzahl dieser Elemente mit umfasst.

Somit wird zunächst ein Kompartment des Dialysators (Blut- oder Dialysatseite) entleert, danach ein Testdruck auf der von Luft oder einem sonstigen Gas gefüllten

Seite aufgebaut (die intakte nasse Membranwand ist für Luft undurchlässig) und anschließend wird der Druckverlauf über die Zeit beobachtet. Aufgrund der Löslichkeit von Gas in Luft kommt es immer zu einem geringfügigen Druckabfall auf der Seite, auf der der Testdruck aufgebracht wurde, bei einem Membranleck ist jedoch der Druckabfall deutlich größer und/oder es kommt zu einem Übertritt von Luftblasen in die nicht entleerte Seite. Diese Bläschen kann man dann am Blasendetektor vorbeiführen und basierend darauf oder basierend auf dem Druckverlauf auf eine Leckage der Membran schließen.

Der Übertritt der Bläschen über die defekte Membran des Dialysators führt zu einem ein Absinken des Luftdruckes im luft- bzw. gasgefüllten, unter Druck stehenden Kompartiment bzw. in dem damit in Verbindung stehenden Leitungssystem und zu einem Druckanstieg auf der flüssigkeitsgefüllten Seite.

Mit dieser Vorgehensweise lassen sich auch Microlecks in der Dialysatormembran mit einem Durchmesser µm-Bereich und größer feststellen. Eine intakte Dialysatormembran hat Poren im nm-Bereich, so dass sich im Druckverhalten ein erheblicher Unterschied zwischen einer intakten und einer defekten Membran ergibt. So lassen sich auch noch Mikrolecks mit einem Durchmesser unterhalb des µm-Bereichs feststellen. Dies können beispielsweise Durchmesser von mehreren Zehntel µm sein.

Der Test funktioniert nur für einen begrenzten Zeitraum, weil sich der Druckgradient abbaut. Allerdings ist der Unterschied im Druckverlauf zwischen einer defekten und einer intakten Membran sehr groß, sodass vorzugsweise eine Beobachtungszeit < 20 s ausreicht.

In einer bevorzugten Ausgestaltung der Erfindung ist vorgesehen, dass im Anschluss an die Überprüfung keine Desinfizierung des dialysatseitigen Leitungssystems vorgenommen wird, sofern bei der Überprüfung kein Leck in der Membran des Dialysators festgestellt wurde. Ein wesentlicher Vorteil der Erfindung besteht somit darin, dass in diesem Fall auf die bislang notwendige Desinfizierung des dialysatseitigen Fluidsystems verzichtet werden kann.

Grundsätzlich kann die Messung des Drucks in der Dialysatkammer, in dem dialysatseitigen Leitungssystem, in der Blutkammer oder in dem blutseitigen Leitungssystem oder in mehreren der vorgenannten Positionen vorgenommen werden.

Die Dialysatkammer und/oder das mit dieser in Fluidverbindung stehende Leitungssystem wird im Folgenden auch als "Dialysatseite" und die Blutkammer und/oder das mit dieser in Fluidverbindung stehende Leitungssystem wird im Folgenden auch als "Blutseite" bezeichnet.

In einer Ausgestaltung der Offenbarung ist vorgesehen, dass in dem blutseitigen Leitungssystem und/oder in dem dialysatseitigen Leitungssystem wenigstens ein Blasendetektor angeordnet ist, der ausgebildet ist, um Luftblasen in dem Blut oder in der Dialysierflüssigkeit zu messen und dass die Überprüfung auf ein Leck der Membran auf der Grundlage der Anzahl der Luftblasen oder einer damit korrelierten Größe vorgenommen wird. Wird auf der flüssigkeitsentleerten Seite ein Testdruck mit Luft oder einem sonstigen Gas aufgebracht und weist die Dialysatormembran ein Leck auf, kommt es zu einem Übertritt der Luft bzw. des Gases in die nicht entleerte Blut- oder Dialysatseite und damit zum Entstehen von Luftblasen auf dieser Seite des Systems.

Um diese erfassen zu können, kann ein Blasendetektor eingesetzt werden, der die Anzahl der an diesem vorbeigeführten Luftblasen pro Zeiteinheit erfasst. Dazu kann die Blutpumpe bzw. die Dialysatpumpe in Betrieb gesetzt werden, um eine Bewegung des Blutes bzw. der Dialysierflüssigkeit und damit auch der darin befindlichen Luftblasen zu erreichen.

Denkbar ist es, dass der Aufbau des Testdruckes mehrfach durchgeführt wird und erst dann mit Schritt c) fortgefahren wird, wenn der Abfall des Testdruckes über die Zeit einen Grenzwert nicht übersteigt. Bei dieser nicht erfindungsgemäßen Vorgehensweise wird zunächst die Blutseite oder die Dialysatseite entleert und sodann auf der entleerten Blut- bzw. Dialysatseite ein Testdruck aufgebracht und der Druckverlauf gemessen. Während dieser Zeit ist die nicht entleerte Seite (Blutseite oder Dialysatseite) nicht geschlossen, sondern z.B. mit einem Ablauf verbunden. Übersteigt der Druckabfall pro Zeiteinheit einen Grenzwert, wird erneut ein Testdruck aufgebaut und der Druckabfall über die Zeit gemessen. Erst wenn dieser unter einem Grenzwert bleibt, wird das Verfahren mit Schritt c) fortgesetzt.

Vorzugsweise erfolgt der Aufbau des Testdruckes mittels eines Kompressors, der Umgebungsluft in die entleerte bzw. zu entleerende Blutseite oder Dialysatseite fördert.

Offenbarungsgemäß kann vorgesehen sein, dass der Druckverlauf oder die Anzahl der detektierten Blasen oder die damit korrelierte Größe angezeigt wird und/oder dass basierend auf einer oder mehreren dieser Messwerte eine Auswertung dahingehend vorgenommen wird, ob die Membran eine Leckage aufweist und das Ergebnis der Auswertung angezeigt wird. Im letzten Fall kann dem Nutzer durch eine Anzeige sofort signalisiert werden, ob der zuletzt eingesetzte Dialysator intakt war und somit auf eine Desinfektion der Dialysatseite verzichtet werden kann.

Die vorliegende Erfindung betrifft des Weiteren eine Vorrichtung zur Überprüfung eines Dialysators auf das Vorhandensein eines Membranlecks, umfassend den Dialysator, der durch wenigstens eine semipermeable Membran in wenigstens eine Blutkammer und in wenigstens eine Dialysatkammer getrennt ist, umfassend wenigstens ein mit der Blutkammer in Fluidverbindung stehendes blutseitiges Leitungssystem, das während der Behandlung mit der Blutkammer sowie mit dem Gefäßsystem eines Patienten verbunden ist, und umfassend wenigstens ein mit der Dialysatkammer in Fluidverbindung stehendes dialysatseitiges Leitungssystem, wobei während der Blutbehandlung, d.h. während des Betriebs des Dialysegerätes das Blut das blutseitige Leitungssystem und die Blutkammer und die Dialysierflüssigkeit die Dialysatkammer und das Dialysierflüssigkeitssystem durchströmt, wobei die Vorrichtung zumindest eine Steuereinheit sowie von dieser Einheit angesteuerte Mittel aufweist, die ausgebildet sind, um die Verfahrensschritte gemäß einem der Ansprüche 1 bis 7 durchzuführen.

Bei diesen Mitteln handelt es sich vorzugsweise um ein oder mehrere Ventile, Pumpen und/oder Kompressoren.

Denkbar ist es, dass die Vorrichtung einen oder mehrere Drucksensoren aufweist, die an der Dialysatkammer, an dem dialysatseitigen Leitungssystem, an der Blutkammer oder an dem blutseitigen Leitungssystem oder in mehreren der vorgenannten Elemente angeordnet sind und dass die Vorrichtung vorzugsweise ausgebildet ist, um den Druckverlauf über die Zeit zu erfassen und basierend darauf auf das Vorhandensein eines Lecks des Dialysators zu schließen.

Dazu kann die Vorrichtung eine Anzeigeeinheit und/oder Auswerteeinheit aufweisen, die den Druckverlauf über die Zeit anzeigt und/oder dahingehend auswertet, ob dieser in einem akzeptablen Bereich liegt oder nicht. Entsprechendes gilt für das Vorhandensein bzw. die Anzahl der detektierten Luftblasen.

Die Vorrichtung kann wenigstens einen Blasendetektor in dem blutseitigen Leitungssystem und/oder in dem dialysatseitigen Leitungssystem aufweisen, der ausgebildet ist, um Luftblasen in der Flüssigkeit (Blut oder Dialysierflüssigkeit) zu messen. Weiterhin kann die Vorrichtung Mittel aufweisen, die derart ausgebildet ist, dass diese die Überprüfung der Dichtigkeit der Membran des Dialysators auf der Grundlage der Anzahl der Luftblasen oder einer damit korrelierten Größe vornehmen. Die Vorrichtung kann Mittel, insbesondere eine oder mehrere Pumpen und/oder Kompressoren, zum Entleeren des Blutes aus der Blutkammer und/oder zum Entleeren der Dialysierflüssigkeit aus der Dialysatkammer aufweisen. Des Weiteren können Mittel, insbesondere wenigstens ein Kompressor, zum Einbringen von unter Druck stehender Luft in die von Blut entleerte Blutkammer bzw. in die Blutseite oder in die von Dialysierflüssigkeit entleerte Dialysatkammer bzw. in die Dialysatseite vorgesehen sein.

Die vorliegende Erfindung betrifft des Weiteren ein Dialysegerät, vorzugsweise ein Gerät zur Durchführung einer Hämodialyse, Hämofiltration oder Hämodiafiltration, wobei das Gerät wenigstens eine Vorrichtung gemäß einem der Ansprüche 8 bis 11 aufweist.

Weitere Einzelheiten und Vorteile der Erfindung werden anhand eines in der Zeichnung dargestellten Ausführungsbeispiels näher erläutert. Es zeigen:
Figur 1: eine schematische Ansicht der Dialysatseite und der Blutseite eines Dialysegerätes,
Figur 2, 3: Druckverläufe über die Zeit für eine intakte und für eine defekte Dialysatormembran.

Das Bezugszeichen D kennzeichnet den Dialysator, der durch eine semipermeable Membran 11, die vorzugsweise durch ein Bündel von Hohlfasern gebildet wird, in eine Dialysatkammer 10 und in eine Blutkammer 12 unterteilt ist.

Mit der Blutkammer 12 steht das blutseitige Leitungssystem BS in Verbindung, das den extrakorporalen Blutkreislauf bildet. In dem blutseitigen Leitungssystem BS befinden sich stromaufwärts des Dialysators D die Blutpumpe 40 und stromaufwärts der Blutpumpe 40 die Klemme K2. Stromabwärts des Dialysators D befindet sich die venöse Tropfkammer 30. Von dieser stromabwärts ist die venöse Klemme K1 angeordnet.

Die Begriffe "stromaufwärts" und "stromabwärts" beziehen sich auf die Strömungsrichtung des Blutes bei in Betrieb befindlicher Blutpumpe 40, die in der Figur durch einen Pfeil markiert ist.

In die Tropfkammer 30 mündet nicht nur die Schlauchleitung, die einen Bestandteil des blutseitigen Leitungssystems BS darstellt, sondern auch eine weitere Leitung 22. In dieser weiteren Leitung 22 befindet sich der Kompressor 20, der zur Durchführung des Verfahrens Luft aus der Umgebung in das blutseitige Leitungssystem BS fördert. Zwischen dem Kompressor 20 und der Tropfkammer 30 befindet sich das Ventil V3. Das Bezugszeichen PV kennzeichnet einen Drucksensor, der sich im venösen Teil des blutseitigen Leitungssystems BS befindet.

Die Bezugszeichen 1 und 2 kennzeichnen den arteriellen und den venösen Konnektor des extrakorporalen Kreislaufs, mit denen dieser mit dem Gefäßsystem des Patienten P verbunden bzw. verbindbar ist.

Im Betrieb der Blutpumpe 40 wird bei konnektiertem Patienten Blut durch die Leitung 41 zum Dialysator D und von dem Dialysator D durch die Leitung 42 und die Tropfkammer 30 zurück zum Patienten gefördert.

Auf der Dialysatseite befindet sich das dialysatseitige Leitungssystem DS, das Leitungen, insbesondere Schlauchleitungen aufweist, mittels derer im Betrieb des Geräts Dialysierflüssigkeit in die bzw. aus der Dialysatkammer 10 geführt wird. Die Bewegung der Dialysierflüssigkeit im dialysatseitigen Leitungssystem DS wird durch eine nicht dargestellte Pumpe vorgenommen.

Die Begriffe "stromaufwärts" und "stromabwärts" beziehen sich auf die Strömungsrichtung der Dialysierflüssigkeit bei in Betrieb befindlicher Pumpe für die Dialysierflüssigkeit, die in der Figur durch einen Pfeil markiert ist. Durch die Leitung 51 wird frische Dialysierflüssigkeit zum Dialysator D gefördert. Verbrauchte Dialysierflüssigkeit wird durch die Leitung 52 vom Dialysator D abgezogen.

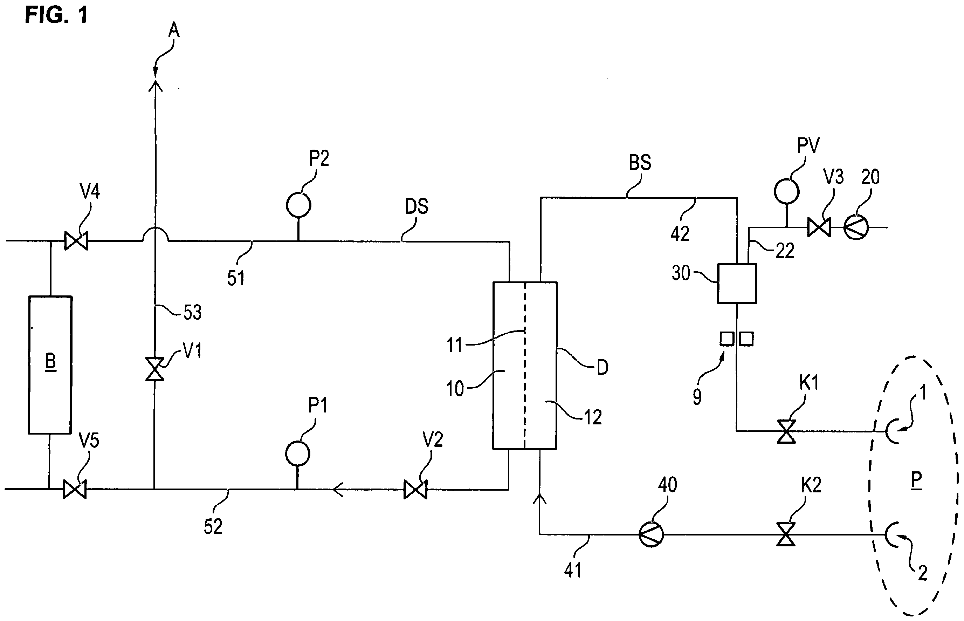

Stromaufwärts und stromabwärts des Dialysators befindet sich in dem dialysatseitigen Leitungssystem DS je ein Drucksensor P1 und P2, wie dies aus der Figur 1 hervorgeht.

Das Bezugszeichen B kennzeichnet schematisch das Bilanziersystem, das zur Bilanzierung der dem Dialysator D zu- und abgeführten Dialysierflüssigkeit dient und mit den zu- und abführenden Leitungen 51 und 52 in Verbindung steht. Die Leitungen 51 und 52 bzw. das Bilanziersystem sind durch Ventile V4, V5 absperrbar.

Von der vom Dialysator D abführenden Leitung 52 zweigt die Leitung 53 ab, die zum Abfluss A für verbrauchte Dialysierflüssigkeit führt.

Wie dies aus der Figur 1 ersichtlich ist, befindet sich zwischen dem Dialysator D und der Abzweigung der Leitung 53 in der Leitung 52 das Ventil V2, mittels dessen die Leitung 52 verschließbar ist. Ein weiteres Ventil V1 ist in der Leitung 53 angeordnet. Mittels des Ventils V1 ist die Leitung 53 absperrbar.

Anstelle der oben genannten Klemmen K1, K2 können auch Ventile oder auch beliebige andere Absperrmittel verwendet werden. Auch die Position der Klemmen ist exemplarisch, d.h. die Klemmen können auch anderer Stelle oder in anderer Anzahl angeordnet sein.

Anstelle der oben genannten Ventile V1, V2, V3, V4, V5 können auch Klemmen oder auch beliebige andere Absperrmittel verwendet werden. Auch die Position der Ventile ist exemplarisch, d.h. die Ventile können auch an anderer Stelle oder in anderer Anzahl angeordnet sein.

Im Folgenden werden exemplarisch zwei Testsequenzen beschrieben, mittels derer ermittelbar ist, ob die Membran 11 eine oder mehrere Leckagen aufweist oder intakt ist.

In einem ersten Ausführungsbeispiel wird zunächst die Blutseite von Blut entleert.

Die Klemmen K1 und K2 sind geschlossen. Die Blutpumpe 40 ist ausgeschaltet. Das Bilanzkammersystem B ist durch Schließen der Ventile V4, V5 abgesperrt. Dies gilt in diesem Ausführungsbeispiel für das gesamte Testverfahren.

Der Kompressor 20 wird eingeschaltet, das Ventil V3 geöffnet und die Ventile V1 und V2 werden geöffnet.

Das auf der Blutseite befindliche Blut wird durch die Luft über die Membran 11 auf die Dialysatseite verdrängt. Es gelangt von dort aus bei geöffneten Ventilen V1, V2 durch die Leitungen 52 und 53 in den Ablauf A.

Der Kompressor bleibt solange in Betrieb, bis mittels des Drucksensors PV ein Druckanstieg festgestellt wird.

Sodann wird der Kompressor 20 ausgeschaltet und das Ventil V1 wird geschlossen.

Im Anschluss daran beginnt der Aufbau des Testdruckes auf der von Blut entleerten Blutseite. Dazu' bleibt die Blutpumpe 40 ausgeschaltet und das Bilanzkammersystem B abgesperrt. Der Kompressor 20 wird eingeschaltet, während das Ventil V1 geschlossen und das Ventil V2 geöffnet bleibt. Der Kompressor 20 bleibt solange eingeschaltet, bis blutseitig am Drucksensor PV ein bestimmter Druck, z.B. 1250 mm Hg erreicht wird.

Sodann wird der Kompressor ausgeschaltet, das Ventil V3 geschlossen und das Ventil V1 wird geöffnet. Sodann wird geprüft, ob der Druckabfall pro Zeit auf der nunmehr belüfteten bzw. mit unter Druck stehender Luft gefüllten Blutseite einen Grenzwert übersteigt. Ist dies der Fall, wird die Prozedur wiederholt, d.h. der Kompressor 20 wird eingeschaltet und das Ventil V3 wieder geöffnet. Das Ventil V1 ist geschlossen und das Ventil V2 ist geöffnet. Sodann wird der Kompressor ausgeschaltet, das Ventil V3 geschlossen und das Ventil V1 wird geöffnet. Anschließend wird der Druckverlauf über die Zeit an dem Drucksensor PV ermittelt.

Dieser Prozess wird wiederholt, bis der Druckabfall pro Zeit auf der Blutseite einen Grenzwert nicht übersteigt.

Sofern dies der Fall ist, beginnt der eigentliche Druckhaltetest. Dazu wird der Kompressor 20 ausgeschaltet, das Ventil V3 geschlossen und das Ventil V1 geschlossen. Das Bilanzkammersystem B bleibt abgesperrt. Der Druckhaltetest wird somit durchgeführt, wenn die mit Dialysierflüssigkeit gefüllte Dialysatseite geschlossen ist, d.h. wenn Zufuhr- und Abfuhrleitungen der Dialysatseite gesperrt sind. Dies erlaubt es, einen Lufteintrag in die Dialysatseite durch einen Druckanstieg auf der Dialysatseite genau zu messen.

Nach dem Abschalten des Kompressors 20 und dem Schließen des Ventils V1 wird der Druckverlauf auf der Dialysatseite mittels des oder der Drucksensoren P1 und/oder P2 ermittelt. Der Filter D wird als in Ordnung qualifiziert, wenn der durch den oder die Sensoren P1 und P2 gemessene Druckanstieg über die Zeit einen Grenzwert nicht übersteigt, d.h. wenn der dialysatseitig gemessene Druckanstieg vergleichsweise gering ist.

Ist dies jedoch nicht der Fall, d.h. steigt der Druck an den Sensoren P1 und/oder P2 vergleichsweise schnell an, ist dies auf eine Leckage der Filtermembran 11 zurückzuführen. Aus der Geschwindigkeit des dialysatseitig gemessenen Druckanstieges kann somit darauf geschlossen werden, ob die Membran des Dialysators D in Ordnung ist oder eine oder mehrere Leckagen aufweist.

Ist der mittels der Sensoren P1 und P2 gemessene Druckanstieg nicht identisch, deutet dies auf einen Lufteintrag in die Hydraulik hin, so dass eine hydrostatische Druckdifferenz durch Lufteintrag zu dieser Abweichung führt.

Alternativ oder zusätzlich zur Messung des Druckanstiegs auf der Dialysatseite ist während des Druckhaltetests auch die Messung des Druckabfalls auf der Blutseite denkbar, obgleich nicht erfindungsgemäß.

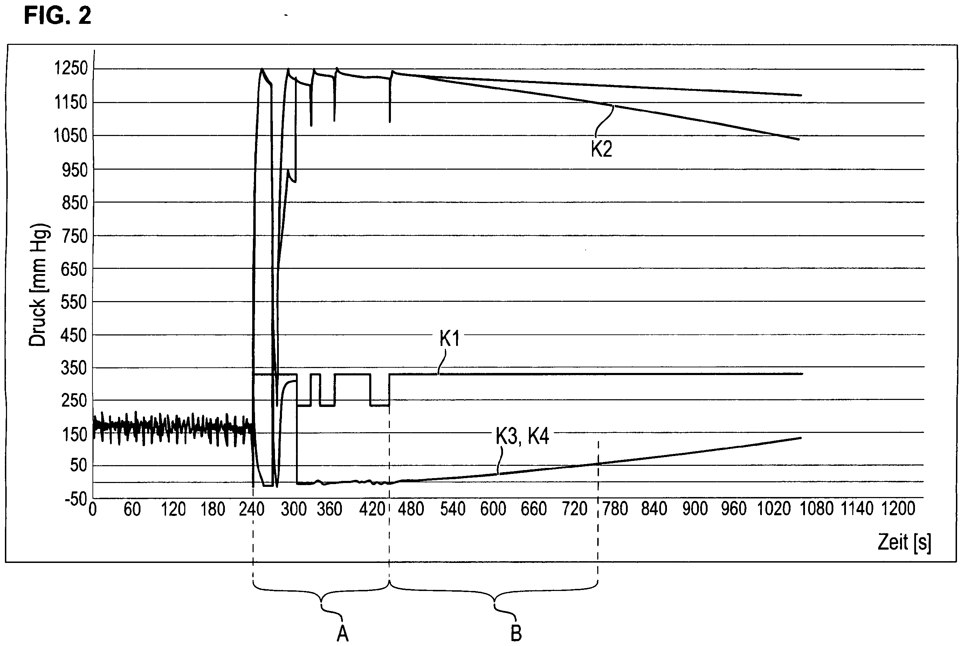

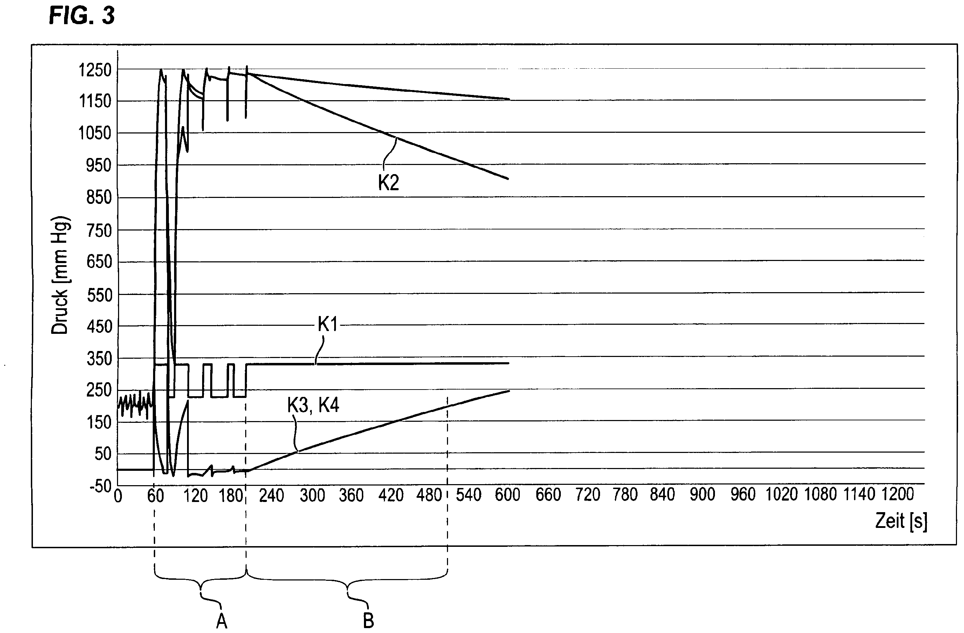

Figur 2 zeigt das Ergebnis des Druckverlaufes über die Zeit für eine intakte Membran und Figur 3 für einen defekten Filter, bei dem die Membran ein oder mehrere Lecks aufweist.

In der Zeitspanne A erfolgt der oben beschriebene Aufbau des Testdruckes auf der Blutseite durch wiederholtes öffnen und Schließen des Ventils V1, was durch den Verlauf K1 gezeigt ist. Der jeweils höhere Wert zeigt das geschlossene Ventil V1, der jeweils geringere Wert zeigt das geöffnete Ventil V1.

Die Kurve K2 zeigt den an dem Drucksensor PV gemessenen Druckverlauf. Wie dies aus Figur 2 ersichtlich ist, wird der Druckaufbau mit Luft auf der entleerten Blutseite so oft wiederholt, bis der Druckverlust bei geöffnetem Ventil V1 und V2 einen bestimmten Grenzwert pro Zeiteinheit nicht übersteigt.

Erst wenn diese Voraussetzung erfüllt ist, startet mit Beginn des Abschnittes B der Druckhaltetest. Dieser wird durchgeführt, während die Dialysatseite gegenüber dem Ablauf A und insgesamt geschlossen ist, so dass der Dialysatseite weder Dialysierflüssigkeit zu- noch abgeführt werden kann.

Die Kurven K3 und K4 zeigen die an den Sensoren P1 und P2 gemessenen Druckverläufe über die Zeit. Aus Figur 2 ergibt sich, dass der Druckanstieg im Bereich von ca. 50 mm Hg in ca. 5 min ab Beginn des Abschnitts B liegt. Dies ist in dem hier beschriebenen Ausführungsbeispiel ein akzeptabler Wert, so dass daraus auf eine integre, d.h. leckfreie Membran geschlossen werden kann.

Demgegenüber liegt bei dem Messergebnis gemäß Figur 3 der Druckanstieg, der an den Sensoren P1 und P2 gemessen wird, in einem Bereich von ca. 200 mm Hg in ca. 5 min ab Beginn des Abschnittes B, was oberhalb eines Grenzwertes liegt. In diesem Fall wird auf eine defekte Membran geschlossen.

Selbstverständlich handelt es sich bei den genannten Werten nur um Beispiele, die die Erfindung nicht beschränken.

Das zweite, nicht erfindungsgemäße Ausführungsbeispiel beginnt mit dem Abklemmen des Patienten durch Schließen der Klemmen K1 und K2 und dem Stoppen der Blutpumpe 40. Der Kompressor 20 ist abgeschaltet und das Ventil V3 geschlossen, so dass die Blutseite insgesamt geschlossen ist.

Sodann folgt die Entleerung der Dialysatseite mittels eines Standard-Entleerungsprogramms durch die Leitung 52 bei geöffneten Ventilen V1 und V2 zum Ablauf A. Zum Entleeren kann ein Belüftungsventil oder der Kompressor in Leitung 51 vorgesehen sein. Beim Ablauf des Entleerungsprogramms ist die Dialysatkupplung offen.

Anschließend wird das Ventil V2 geschlossen und es wird auf der von Dialysierflüssigkeit entleerten Dialysatseite ein Testdruck mittels Luft oder einem sonstigen Gas aufgebaut. Dieser Druckaufbau erfolgt mittels eines Kompressors, bis der gewünschte Testdruck, z.B. 1250 mm Hg erreicht ist. Der Kompressor wird gestoppt.

Sobald dies der Fall ist, wird das Ventil V2 geschlossen und die Konnektoren 1 und 2 miteinander verbunden, so dass sich ein geschlossener Blutkreislauf bildet. Die Blutpumpe 40 wird in Betrieb genommen.

Der Luftblasendetektor 9, der beispielsweise stromabwärts der Tropfkammer 30 angeordnet ist, erfasst die Anzahl der Luftblasen pro Zeiteinheit bei laufender Blutpumpe 40. Die Membran 11 wird als intakt qualifiziert, wenn die gezählte Anzahl von Luftblasen pro Zeiteinheit einen Grenzwert nicht erreicht, andernfalls wird auf eine defekte Membran geschlossen.

Alternativ oder zusätzlich wird der Druck an den dialysatseitigen Drucksensoren P1 und P2 gemessen. Denkbar ist es, dass das Ergebnis dieser Druckmessung als auch der Messwert des Luftblasendetektors zur Bestimmung der Integrität der Membran 11 herangezogen wird.

So ist es beispielsweise möglich, dass nur dann auf eine integre Membran geschlossen wird, wenn die Anzahl der Luftblasen pro Zeiteinheit unterhalb eines Grenzwertes bleibt und zusätzlich der Druckverlust auf der Dialysatseite einen Grenzwert nicht übersteigt.

Mittels der vorliegenden Erfindung lassen sich Mikrolecks in einem Dialysator erkennen. Das Verfahren erfolgt nach dem Abklemmen des Patienten und nach der Durchführung der Blutbehandlung. Durch das Verfahren lassen sich auch ohne nachfolgende Desinfizierung der Dialysatseite Kreuzkontaminationen vermeiden.

## Patentansprüche

1. Verfahren zur Überprüfung eines Dialysators (D) auf das Vorhandensein eines Lecks in der semipermeablen Membran (11) des Dialysators (D), wobei die Membran (11) den Dialysatorinnenraum in wenigstens eine Blutkammer (12) und in wenigstens eine Dialysatkammer (10) unterteilt, wobei die Blutkammer (12) im Betrieb des Dialysators von Blut durchströmt wird und mit einem blutseitigen Leitungssystem (BS) und dem Gefäßsystem des Patienten (P) in Fluidverbindung steht und wobei die Dialysatkammer (10) im Betrieb des Dialysators von Dialysierflüssigkeit durchströmt wird und mit einem dialysatseitigen Leitungssystem (DS) in Fluidverbindung steht, wobei das Verfahren die folgenden Schritte umfasst:
a) Entleeren der Blutkammer (12) oder der Dialysatkammer (10) von Blut bzw. von Dialysierflüssigkeit und Beibehalten der Flüssigkeit (Blut oder Dialysat) in der nicht entleerten Dialysat- oder Blutkammer (10, 12),
b) Aufbauen eines Testdruckes mittels eines Gases, insbesondere mittels Luft in der entleerten Blutkammer (12) oder in der entleerten Dialysatkammer (10) und
c) Messen des Druckanstiegs in der nicht entleerten Blutkammer (12) oder in der nicht entleerten Dialysatkammer (10) oder in dem mit diesen jeweils in Fluidverbindung stehenden Leitungssystem (BS, DS),
wobei das gesamte Verfahren, einschließlich die Schritte a) bis c) im Anschluss an die Blutbehandlung des Patienten (P) und im Anschluss an das Abklemmen des Patienten (P) von dem blutseitigen Leitungssystem (BS) durchgeführt werden.

2. Verfahren nach Anspruch 1, **dadurch gekennzeichnet, dass** im Anschluss an die Überprüfung gemäß der Schritte a) bis c) keine Desinfizierung des dialysatseitigen Leitungssystems (DS) vorgenommen wird, sofern bei der Überprüfung kein Leck in der Membran (11) des Dialysators (D) festgestellt wurde.

3. Verfahren nach Anspruch 1 oder 2, **dadurch gekennzeichnet, dass** die Messung des Drucks in der Dialysatkammer (10), in dem dialysatseitigen Leitungssystem (DS), in der Blutkammer (12) oder in dem blutseitigen Leitungssystem (BS) oder in mehreren der vorgenannten Elemente vorgenommen wird.

4. Verfahren nach einem der vorhergehenden Ansprüche, **dadurch gekennzeichnet, dass** Schritt c) durchgeführt wird, während die nicht entleerte Kammer (10, 12) bzw. das nicht entleerte Leitungssystem (BS, DS) geschlossen ist.

5. Verfahren nach einem der vorhergehenden Ansprüche, **dadurch gekennzeichnet, dass** der Aufbau des Testdruckes mehrfach durchgeführt wird und erst dann mit Schritt c) fortgefahren wird, wenn der Abfall des Testdruckes über die Zeit nicht einen Grenzwert übersteigt.

6. Verfahren nach einem der vorhergehenden Ansprüche, **dadurch gekennzeichnet, dass** der Aufbau des Testdruckes in Schritt b) mittels eines Kompressors durchgeführt wird, der Umgebungsluft in die entleerte Blutkammer (12) oder Dialysatkammer (10) bzw. in das mit diesen in Verbindung stehende Leitungssystem (LS, DS) fördert.

7. Verfahren nach einem der vorhergehenden Ansprüche, **dadurch gekennzeichnet, dass** der Druckverlauf angezeigt wird und/oder dass basierend auf einer oder mehreren dieser Messwerte eine Auswertung dahingehend vorgenommen wird, ob die Membran (11) eine Leckage aufweist und das Ergebnis der Auswertung angezeigt wird.

8. Vorrichtung zur Überprüfung eines Dialysators (D) auf das Vorhandensein eines Lecks in der Membran (11) des Dialysators (D), umfassend den Dialysator (D), der durch die wenigstens eine semipermeable Membran (11) in wenigstens eine Blutkammer (12) und in wenigstens eine Dialysatkammer (10) getrennt ist, umfassend wenigstens ein mit der Blutkammer (12) in Fluidverbindung stehendes blutseitiges Leitungssystem (BS), das während der Behandlung mit der Blutkammer (12) sowie mit dem Gefäßsystem eines Patienten (P) in Fluidverbindung steht, und umfassend wenigstens ein mit der Dialysatkammer (10) in Fluidverbindung stehendes dialysatseitiges Leitungssystem (DS), wobei während der Blutbehandlung das Blut das blutseitige Leitungssystem (BS) und die Blutkammer (12) und die Dialysierflüssigkeit die Dialysatkammer (10) und das Dialysierflüssigkeitssystem (DS) durchströmt,
wobei die Vorrichtung eine Steuereinheit sowie von dieser Einheit angesteuerte Mittel aufweist, die ausgebildet sind, um die Verfahrensschritte gemäß einem der Ansprüche 1 bis 7 durchzuführen.

9. Vorrichtung nach Anspruch 8, **dadurch gekennzeichnet, dass** die Vorrichtung einen oder mehrere Drucksensoren aufweist, die an der Dialysatkammer (10), an dem dialysatseitigen Leitungssystem (DS), an der Blutkammer (12) oder an dem blutseitigen Leitungssystem (BS) oder in mehreren der vorgenannten Elemente unmittelbar oder mittelbar angeordnet sind und dass die Vorrichtung ausgebildet ist, um den Druckverlauf über die Zeit zu erfassen und vorzugsweise basierend darauf auf das Vorhandensein eines Lecks in der Membran (11) des Dialysators (D) zu schließen.

10. Vorrichtung nach einem der Ansprüche 8 bis 9, **dadurch gekennzeichnet, dass** die Vorrichtung Mittel zum Entleeren des Blutes aus der Blutkammer (12) und/oder zum Entleeren der Dialysierflüssigkeit aus der Dialysatkammer (10) aufweist und dass die Vorrichtung Mittel, insbesondere wenigstens einen Kompressor, zum Einbringen von unter Druck stehender Luft in die von Blut entleerte Blutkammer (12) oder in die von Dialysierflüssigkeit entleerte Dialysatkammer (10) und ggf. das mit diesen in Fluidverbindung stehende Leitungssystem (LS, DS) umfasst.

11. Vorrichtung nach einem der Ansprüche 8 bis 10, **dadurch gekennzeichnet, dass** die Vorrichtung Anzeigemittel aufweist, die ausgebildet sind, den Druckverlauf anzuzeigen und/oder dass die Vorrichtung eine Auswerteeinheit aufweist, die ausgebildet ist, dass diese basierend auf einer oder mehreren dieser Messwerte eine Auswertung dahingehend vornimmt, ob die Membran (11) eine Leckage aufweist und das Ergebnis der Auswertung anzeigt.

12. Dialysegerät, **dadurch gekennzeichnet, dass** das Dialysegerät wenigstens eine Vorrichtung gemäß einem der Ansprüche 8 bis 11 aufweist.

## Claims

1. Method for checking a dialyzer (D) for the presence of a leak in the semipermeable membrane (11) of the dialyzer (D), wherein the membrane (11) divides the inner dialyzer space into a least one blood chamber (12) and into at least one dialyzate chamber (10), wherein the blood chamber (12) is flowed through by blood in the operation of the dialyzer and is in fluid communication with a blood-side line system (BS) and the vascular system of the patient (P) and wherein the dialyzate chamber (10) is flowed through by dialysis fluid in the operation of the dialyzer and is in fluid communication with a dialyzate-side line system (DS), wherein the method comprises the following steps:
a) emptying the blood chamber (12) or the dialyzate chamber (10) of blood and of dialysis fluid respectively, and keeping the fluid (blood or dialyzate) in the non-emptied dialyzate chamber or blood chamber (10, 12),
b) building up a test pressure by means of a gas, in particular by means of air, in the emptied blood chamber (12) or in the emptied dialyzate chamber (10), and
c) measuring the pressure increase in the non-emptied blood chamber (12) or in the non-emptied dialyzate chamber (10) or in the line system (BS, DS) respectively in fluid communication therewith
wherein the entire method including the steps a) to c) are carried out subsequent to the blood treatment of the patient (P) and subsequent to the disconnection of the patient (P) from the blood-side line system (BS).

2. Method in accordance with claim 1, **characterized in that**, subsequent to the check in accordance with steps a) to c), no disinfection of the dialyzate-side line system (DS) is carried out if no leak was detected in the membrane (11) of the dialyzer (D) on the check.

3. Method in accordance with claim 1 or claim 2, **characterized in that** the measurement of the pressure is carried out in the dialyzate chamber (10), in the dialyzate-side line system (DS), in the blood chamber (12) or in the blood-side line system (BS) or in a plurality of the aforesaid elements.

4. Method in accordance with one of the preceding claims, **characterized in that** step c) is carried out while the non-emptied chamber (10, 12) or the non-emptied line system (BS, DS) is closed.

5. Method in accordance with one of the preceding claims, **characterized in that** the build-up of the test pressure is carried out multiple times and a continuation is only made with step c) when the drop of the test pressure over time does not exceed a limit value.

6. Method in accordance with one of the preceding claims, **characterized in that** the build-up of the test pressure in step b) is carried out by means of a compressor which conveys environmental air into the emptied blood chamber (12) or dialyzate chamber (10) or in the line system (LS, DS) in communication therewith.

7. Method in accordance with one of the preceding claims, **characterized in that** the pressure development is displayed and/or that an evaluation is made on the basis of one or more of these measured values as to whether the membrane (11) has a leak and the result of the evaluation is displayed.

8. Apparatus for checking a dialyzer (D) for the presence of a leak in the membrane (11) of the dialyzer (D), comprising the dialyzer (D) which is divided by the at least one semipermeable membrane (11) into at least one blood chamber (12) and into at least one dialyzate chamber (10), comprising at least one blood-side line system (BS) which is in fluid communication with the blood chamber (12) and which is in fluid communication with the blood chamber (12) and with the vascular system of a patient (P) during the treatment, and comprising at least one dialyzate-side line system (DS) in fluid communication with the dialyzate chamber (10), wherein the blood flows through the blood-side line system (BS) and the blood chamber (12) and the dialysis fluid flows through the dialyzate chamber (10) and the dialysis fluid system (DS) during the blood treatment, wherein the apparatus comprises a control unit and means which are controlled by this unit and are configured to carry out the method steps in accordance with one of the claims 1 to 7.

9. Apparatus in accordance with claim 8, **characterized in that** the apparatus comprises one or more pressure sensors which are arranged indirectly or directly at the dialyzate chamber (10), at the dialyzate-side line system (DS), at the blood chamber (12) or at the blood-side line system (BS) or in a plurality of the aforesaid elements, and **in that** the apparatus is configured to detect the pressure development over time and to draw a conclusion on the presence of a leak in the membrane (11) of the dialyzer (D) preferably on the basis thereof.

10. Apparatus in accordance with one of the claims 8 to 9, **characterized in that** the apparatus comprises means for emptying the blood from the blood chamber (12) and/or for emptying the dialysis fluid from the dialyzate chamber (10), and **in that** the apparatus comprises means, in particular at least one compressor, for the introduction of pressurized air into the blood chamber (12) emptied of blood or into the dialyzate chamber (10) emptied of dialysis fluid and optionally the line system (LS, DS) in fluid communication therewith.

11. Apparatus in accordance with one of the claims 8 to 10, **characterized in that** the apparatus comprises display means which are configured to display the pressure development and/or **in that** the apparatus comprises an evaluation unit which is configured such that it carries out an evaluation on the basis of one or more of said measured values as to whether the membrane (11) has a leak and displays the result of the evaluation.

12. Dialysis device, **characterized in that** the dialysis device comprises at least one apparatus in accordance with one of the claims 8 to 11.

## Revendications

1. Procédé de vérification de la présence d'une fuite sur un dialyseur (D) dans la membrane (11) semi-perméable du dialyseur (D), dans lequel la membrane (11) divise l'espace intérieur de dialyseur en au moins une chambre pour le sang (12) et en au moins une chambre pour le dialysat (10), dans lequel la chambre pour le sang (12) est traversée lors du fonctionnement du dialyseur par du sang et se trouve en communication fluidique avec un système de conduit côté sang (BS) et le système vasculaire du patient (P) et dans lequel la chambre pour le dialysat (10) est traversée lors du fonctionnement du dialyseur par du liquide de dialyse et se trouve en communication fluidique avec un système de conduit côté dialysat (DS), dans lequel le procédé comprend les étapes suivantes :
a) de vidange de la chambre pour le sang (12) ou de la chambre pour le dialysat (10) du sang ou du liquide de dialyse et de maintien du liquide (sang ou dialysat) dans la chambre pour le dialysat ou pour le sang (10, 12) non vidée,
b) d'établissement d'une pression de test au moyen d'un gaz, en particulier au moyen d'air dans la chambre pour le sang (12) vidée ou dans la chambre pour le dialysat (10) vidée, et
c) de mesure de l'augmentation de pression dans la chambre pour le sang (12) non vidée ou dans la chambre pour le dialysat (10) non vidée ou dans le système de conduit (BS, DS) se trouvant respectivement en communication fluidique avec celles-ci,
dans lequel l'ensemble du procédé y compris les étapes a) à c) sont effectués directement après le traitement du sang du patient (P) et directement après le débranchement du patient (P) du système de conduit côté sang (BS).

2. Procédé selon la revendication 1, **caractérisé en ce qu'**aucune désinfection du système de conduit côté dialysat (DS) n'est réalisée directement après la vérification selon les étapes a) à c) dans la mesure où aucune fuite n'a été constatée dans la membrane (11) du dialyseur (D) lors de la vérification.

3. Procédé selon la revendication 1 ou 2, **caractérisé en ce que** la mesure de la pression est réalisée dans la chambre pour le dialysat (10), dans le système de conduit côté dialysat (DS), dans la chambre pour le sang (12) ou dans le système de conduit côté sang (BS) ou dans plusieurs des éléments susmentionnés.

4. Procédé selon l'une quelconque des revendications précédentes, **caractérisé en ce que** l'étape c) est mise en œuvre alors que la chambre (10, 12) non vidée ou le système de conduit (BS, DS) non vidé est fermée ou fermé.

5. Procédé selon l'une quelconque des revendications précédentes, **caractérisé en ce que** l'établissement de la pression de test est mis en œuvre à maintes reprises et se poursuit seulement avec l'étape c) quand la chute de la pression de test ne dépasse pas sur le temps une valeur limite.

6. Procédé selon l'une quelconque des revendications précédentes, **caractérisé en ce que** l'établissement de la pression de test est mise en œuvre lors de l'étape b) au moyen d'un compresseur, qui refoule de l'air ambiant dans la chambre pour le sang (12) ou la chambre pour le dialysat (10) vidée ou dans le système de conduit (LS, DS) relié à celles-ci.

7. Procédé selon l'une quelconque des revendications précédentes, **caractérisé en ce que** l'évolution de pression est affichée et/ou que sur la base d'une ou de plusieurs desdites valeurs de mesure, une évaluation est réalisée pour savoir si la membrane (11) présente une fuite et le résultat de l'évaluation est affiché.

8. Dispositif de vérification de la présence d'une fuite sur un dialyseur (D) dans la membrane (11) du dialyseur (D), comprenant le dialyseur (D), qui est séparé par l'au moins une membrane (11) semi-perméable en au moins une chambre pour le sang (12) et en au moins une chambre pour le dialysat (10), comprenant au moins un système de conduit côté sang (BS) se trouvant en communication fluidique avec la chambre pour le sang (12), qui se trouve en communication fluidique au cours du traitement avec la chambre pour le sang (12) ainsi qu'avec le système vasculaire d'un patient (P), et comprenant au moins un système de conduit côté dialysat (DS) se trouvant en communication fluidique avec la chambre pour le dialysat (10), dans lequel au cours du traitement du sang, le sang traverse le système de conduit côté sang (BS) et la chambre pour le sang (12) et le liquide de dialyse traverse la chambre pour le dialysat (10) et le système de liquide de dialyse (DS), dans lequel le dispositif présente une unité de commande ainsi que des moyens pilotés par ladite unité, lesquels sont réalisés pour mettre en œuvre les étapes de procédé selon l'une quelconque des revendications 1 à 7.

9. Dispositif selon la revendication 8, **caractérisé en ce que** le dispositif présente un ou plusieurs capteurs de pression, qui sont disposés directement ou indirectement au niveau de la chambre pour le dialysat (10), au niveau du système de conduit côté dialysat (DS), au niveau de la chambre pour le sang (12) ou au niveau du système de conduit côté sang (BS) ou dans plusieurs des éléments susmentionnés, et que le dispositif est réalisé pour détecter sur le temps et pour déduire, de préférence sur la base de la présence d'une fuite dans la membrane (11) du dialyseur (D), l'évolution de pression.

10. Dispositif selon l'une quelconque des revendications 8 à 9, **caractérisé en ce que** le dispositif présente des moyens servant à vider le sang hors de la chambre pour le sang (12) et/ou servant à vider le liquide de dialyse hors de la chambre pour le dialysat (10), et que le dispositif comprend des moyens, en particulier au moins un compresseur, servant à introduire de l'air sous pression dans la chambre pour le sang (12) vidée du sang ou dans la chambre pour le dialysat (10) vidée du liquide de dialyse et éventuellement le système de conduit (LS, DS) se trouvant en communication fluidique avec celles-ci.

11. Dispositif selon l'une quelconque des revendications 8 à 10, **caractérisé en ce que** le dispositif présente des moyens d'affichage, qui sont réalisés pour afficher l'évolution de pression, et/ou que le dispositif présente une unité d'analyse, qui est réalisée pour que celle-ci réalise sur la base d'une ou de plusieurs desdites valeurs de mesure une analyse pour savoir si la membrane (11) présente une fuite et pour qu'elle affiche le résultat de l'analyse.

12. Appareil de dialyse, **caractérisé en ce que** l'appareil de dialyse présente au moins un dispositif selon l'une quelconque des revendications 8 à 11.
